Europäisches Patentamt

European Patent Office

Office européen des brevets

(19)

(11) Numéro de publication : **0 539 435 B1**

(12) **FASCICULE DE BREVET EUROPEEN**

(45) Date de publication du fascicule du brevet :
14.12.94 Bulletin 94/50

(51) Int. Cl.⁵ : **G01N 33/52**, G01N 33/58,
G01N 33/542, // G01N33/533

(21) Numéro de dépôt : **91913034.4**

(22) Date de dépôt : **12.07.91**

(86) Numéro de dépôt international :
**PCT/FR91/00566**

(87) Numéro de publication internationale :
**WO 92/01224 23.01.92 Gazette 92/03**

(54) PROCEDE DE REDUCTION D'INTERFERENCES DANS UN DOSAGE PAR FLUORESCENCE.

(30) Priorité : **13.07.90 FR 9008982**

(43) Date de publication de la demande :
**05.05.93 Bulletin 93/18**

(45) Mention de la délivrance du brevet :
**14.12.94 Bulletin 94/50**

(84) Etats contractants désignés :
**AT BE CH DE DK ES FR GB GR IT LI LU NL SE**

(56) Documents cités :
**EP-A- 0 321 353**
**WO-A-90/05302**
**US-A- 4 542 104**
**JOURNAL OF THE AMERICAN CHEMICAL SOCIETY, vol. 102, no. 7, 26 Mars 1980, American Chemical Society, US; E.L. YEE et al., pp. 2278-2285**

(73) Titulaire : **CIS BIO INTERNATIONAL
RN 306
F-91400 Saclay (FR)**

(72) Inventeur : **MATHIS, Gérard
17, impasse de la Capelle-des-Ladres
F-30200 Bagnols-sur-Cèze (FR)**
Inventeur : **DUMONT, Christophe La Chafranière
Saint-André-d'Olerargues
F-30330 Connaux (FR)**
Inventeur : **JOLU, Etienne, Jean-Pierre
2, allée du Romarin
F-30200 Bagnols-sur-Cèze (FR)**

(74) Mandataire : **Gillard, Marie-Louise
Cabinet Beau de Loménie
158, rue de l'Université
F-75340 Paris Cédex 07 (FR)**

## Description

La présente invention a pour objet un procédé de réduction d'interférences dans un dosage par fluorescence.

L'utilisation de dosages immunologiques pour l'analyse qualitative et quantitative de composés dans des fluides biologiques est à l'heure actuelle largement répandue.

Parmi les techniques existantes, les dosages par fluorimétrie ont pris une importance croissante.

En effet, ils présentent un certain nombre d'avantages parmi lesquels la sensibilité, la rapidité de la mesure, la stabilité et l'inocuité des réactifs marqués par des composés fluorescents et le coût relativement réduit.

Il est connu que les méthodes de détection utilisant la fluorescence sont intrinsèquement très sensibles et pourraient permettre des limites de détection inférieures à celles atteintes par des dosages immunologiques utilisant des réactifs radiomarqués, en particulier par l'utilisation de sources lumineuses modulables laser (I. Wieder, Immunofluorescence and related staining techniques, 1978, Elsevier).

De nombreuses molécules fluorescentes utilisables comme traceur dans ce type de dosages ont été précédemment décrites parmi lesquelles les complexes de terre rare possèdent des propriétés intéressantes.

L'utilisation de complexes particuliers, les cryptates de terre rare, est décrite dans les demandes EP 0 180 492, PCT/FR 86 00269, EP 0 321 353 ou PCT/FR 89 00562.

Ces cryptates de terre rare présentent l'avantage d'être très stables en milieu protéique et salin, cette propriété étant particulièrement importante dans le cas des immunoessais homogènes.

La sensibilité de la mesure peut néanmoins être fortement affectée par des interférences de différents types résultant de la présence de diverses molécules dans le milieu dans lequel on effectue la mesure.

Ce problème se pose de manière particulièrement aigüe dans le cas de dosages en milieu sérique dans lequel de nombreuses molécules sont susceptibles d'interférer.

Le signal mesuré peut par exemple être parasité par l'émission de molécules susceptibles d'être excitées et d'émettre aux mêmes longueurs d'onde que la molécule utilisée comme traceur.

Les méthodes de mesure de fluorescence en temps résolu permettent de remédier partiellement à cet inconvénient. Le principe de ces méthodes réside dans le fait qu'on effectue la mesure de la fluorescence émise par une molécule traceur ayant une durée de vie d'émission relativement longue, la mesure étant retardée dans le temps au-delà de la durée de vie d'émission des autres molécules présentes.

Il est dans ce cas nécessaire d'utiliser des molécules fluorescentes traceurs à durée de vie relativement longue telles que les chélates de terre rare.

La sensibilité de la mesure peut également être affectée par l'interférence de molécules du milieu susceptible de perturber la variation de fluorescence résultant de la liaison entre l'analyte à détecter et le réactif biospécifique marqué. La demande EP 0 324 323 décrit l'utilisation d'un modulateur qui stabilise le chélate de terre rare lié au réactif biospécifique, de telle sorte que la fluorescence mesurée soit réellement fonction de la concentration de l'analyte. Ce modulateur a pour effet d'éviter la perturbation de la fluorescence du chélate de terre rare par les autres molécules présentes dans le milieu. La variation de fluorescence mesurée est alors fonction de la seule réaction antigène-anticorps. Les modulateurs proposés sont des macromolécules telles que des protéines et des détergents, et doivent être utilisées en excès dans la gamme de 0,1 à 10 g/l.

Le problème des interférences dues aux molécules présentes dans le milieu de mesure n'est néanmoins complètement résolu par aucune de ces méthodes. En effet, une source importante de limitation de la sensibilité de la mesure par fluorescence est l'existence de processus d'extinction ("quenching") dus à des molécules présentes dans le milieu capables d'inhiber la fluorescence de la molécule fluorescente utilisée comme marqueur dans le dosage. Dans le cas des complexes de terre rares, ces processus peuvent résulter de mécanismes de transfert d'électrons par proximité, dans lesquels la molécule inhibitrice vient occuper les sites de coordination restés libres au sein du complexe. On peut en particulier citer les réactions red/ox intervenant entre la molécule fluorescente et des molécules présentes dans le milieu. Ces mécanismes sont susceptibles de faire varier de façon non négligeable la fluorescence émise.

L'inhibition de fluorescence due aux phénomènes red/ox n'est pas décrite dans la littérature relative aux dosages immunofluorescents homogènes.

L'article Weber et al, Clin. Chem, 1983, 29/9, 1665-1672, décrit l'influence d'interférences dues en particulier à l'acide urique dans la détection ampérométrique d'un complexe Tris(2',2'-bipyridine)ruthenium (III). Ce complexe provient de la réaction red/ox du complexe correspondant Ru(II) capable d'oxyder un complexe extincteur Co(III). L'acide urique a été identifié en tant qu'agent réducteur de Ru(III) et est donc susceptible d'interférer dans la mesure.

Ce mécanisme red/ox dû à un transfert d'électron entre un composé fluorescent et un composé extincteur a également été mis en évidence par Sabbatini et al., J.A.C.S., 1984, 106, 4055-4056. Cet article décrit notamment l'oxydation d'un complexe $M(CN)_6^{4-}$ dans lequel M est le fer, le ruthenium ou l'osmium par un cryptate d'europium.

L'inhibition de la fluorescence par des mécanismes mettant en jeu un transfert d'électrons et en général par des mécanismes d'extinction est un phénomène extrêmement gênant dans la pratique car les éléments inhibiteurs peuvent soit se trouver naturellement présents en tant que composants dans le milieu de mesure (par exemple l'acide urique dans le sérum) ou encore y être ajoutés comme additifs ou stabilisants pour le dosage.

Ces inhibiteurs affectent fortement la fluorescence de la molécule marqueur. En particulier, dans le cas des réactions parasites red/ox le passage de l'état réduit à l'état oxydé d'un ion de terre rare par le biais d'un mécanisme red/ox entraîne une diminution de la durée de vie et une modification de spectre d'émission du complexe le contenant, de telle sorte que la sensibilité de la mesure est fortement affectée.

De manière inattendue, on a maintenant trouvé que l'addition d'ions fluorure dans le milieu de mesure permet de diminuer de façon importante l'inhibition de fluorescence due à la présence de molécules inhibitrices dans ce milieu .

En particulier, ces interférences sont notablement réduites en milieu biologique.

De façon surprenante, il a également été trouvé que ces ions fluorures n'altéraient pas la stabilité des complexes cryptates de terre rare utilisés, contrairement à ce qui était décrit dans Yee et al, J.A.C.S., 1980, 102, 2278-2285, où l'association d'ions fluorures dans un cryptate de terre rare trivalent se traduisait par une accélération importante de la vitesse de dissociation du cryptate, la demi-vie du produit passant de 27 jours à 2,8 jours.

En effet, on a observé qu'en ajoutant au milieu de mesure des ions fluorures, en particulier sous forme de fluorure de sodium, la durée de vie du signal émis par un cryptate ou un chélate de terre rare utilisé comme molécule traceur redevenait comparable à celle du signal mesurée dans le tampon seul.

A titre d'hypothèse, on peut penser que les ions fluorures, de par leur petite taille et leur charge négative, peuvent occuper les sites de fixation libres dans les complexes de terre rare, et ainsi empêcher l'accès d'inhibiteurs.

L'invention a donc pour objet un procédé de réduction d'interférences dans un dosage par fluorescence d'un analyte utilisant comme traceur au moins un composé fluorescent, caractérisé en ce qu'on ajoute au milieu de mesure des ions fluorures.

Dans la présente description on définit par :
- "analyte" toute substance ou groupe de substances analogues à détecter et/ou déterminer ;
- "récepteur" toute substance capable de se fixer spécifiquement sur un site dudit analyte.

Dans un aspect préféré, le milieu de mesure est un milieu biologique, tel qu'un milieu sérique.

De manière avantageuse, on utilisera du fluorure de sodium , de cesium, de potassium ou de lithium lorsque le milieu de mesure est un milieu aqueux. Le choix du cation dépendra du milieu utilisé et sera choisi de manière à ce que les ions fluorures soient solubles dans le milieu, ce choix étant à la portée de l'homme du métier.

En particulier, on utilisera en milieu sérique du fluorure de sodium (NaF) à une concentration de 50 à 500 mM/l.

Dans un autre aspect avantageux, on utilisera du fluorure de potassium (KF) à une concentration de 50 mM/l à 5 M/l.

En tant que composé fluorescent traceur, on utilisera avantageusement un chélate ou un cryptate de terre rare.

En tant qu'ion de terre rare, on peut utiliser en particulier des ions de terbium, europium, dysprosium, samarium ou neodymium. On utilisera de préférence le terbium ou l'europium.

Le procédé selon l'invention est avantageusement utilisable pour réduire les interférences dans le milieu de mesure d'un procédé de détection et/ou de détermination par fluorescence d'un analyte dans un milieu susceptible de le contenir.

Le procédé de l'invention trouve une application importante dans les dosages immunologiques par fluorescence, aussi bien dans les procédés de dosage dites par compétition ou par excès, en phase homogène ou hétérogène, décrits dans l'art antérieur (Landon, Ann. Clin. Biochem, 1981, 18, 253 et E. SOINI et al, Clin. Chem. 1979, 25, 353).

En particulier, le procédé de détection et/ou de détermination de l'analyte est un procédé homogène.

Dans un aspect préféré, le procédé de réduction d'interférences de l'invention est utilisable dans un procédé de détection et/ou de détermination par fluorescence d'un analyte dans un milieu susceptible de le contenir consistant :
1) à ajouter audit milieu un premier réactif constitué d'au moins un récepteur dudit analyte ,
2) à ajouter un second réactif choisi parmi l'analyte ou au moins l'un de ses récepteurs, l'un des deux réactifs étant couplé avec un composé fluorescent donneur et l'autre réactif étant couplé avec un composé fluorescent accepteur et l'ordre d'ajout des réactifs pouvant être inversé,

3) à faire incuber le milieu résultant soit après l'addition de chaque réactif, soit après l'addition des deux réactifs,

4) à exciter le milieu résultant à la longueur d'onde d'excitation du composé donneur,

5) à mesurer, à l'équilibre ou en cinétique, le signal émis par le composé fluorescent accepteur.

Dans un aspect avantageux, on utilisera le procédé de l'invention dans un procédé de détection et/ou de détermination par fluorescence d'un analyte dans un milieu susceptible de le contenir à l'aide d'une méthode par excès consistant :

1) à ajouter, audit milieu contenant l'analyte recherché, un premier réactif constitué par au moins un récepteur dudit analyte, couplé avec un composé fluorescent donneur,

2) à ajouter un second réactif constitué par un ou plusieurs autres récepteurs dudit analyte, ledit second réactif étant couplé avec un composé fluorescent accepteur,

3) à faire incuber ledit milieu après chaque addition de réactifs ou après l'addition des deux réactifs,

4) à exciter le milieu résultant à la longueur d'onde d'excitation du composé fluorescent donneur par une source de lumière,

5) à mesurer le signal émis par le composé fluorescent accepteur.

Dans un aspect préféré, on utilisera dans la méthode par excès ci-dessus un seul récepteur de l'analyte qui est couplé soit avec le composé fluorescent donneur, soit avec le composé fluorescent accepteur.

On peut également utiliser le procédé selon l'invention dans un procédé de détection et/ou de détermination par fluorescence d'un analyte dans un milieu susceptible de le contenir à l'aide d'une méthode par compétition consistant :

1) à ajouter, audit milieu contenant l'analyte recherché, un premier réactif constitué par un récepteur dudit analyte, couplé avec un composé fluorescent donneur,

2) à ajouter un second réactif constitué de l'analyte couplé avec un composé fluorescent accepteur,

3) à faire incuber ledit milieu après chaque addition de réactifs ou après l'addition des deux réactifs,

4) à exciter le milieu résultant à la longueur d'onde d'excitation du composé fluorescent donneur,

5) à mesurer le signal émis par le composé fluorescent accepteur.

Le procédé de réduction d'interférences selon l'invention peut encore être utilisé dans un procédé de détection et/ou de détermination par fluorescence d'un analyte dans un milieu susceptible de le contenir à l'aide d'une méthode par compétition consistant :

1) à ajouter, audit milieu contenant l'analyte recherché, un premier réactif constitué par un récepteur dudit analyte, ledit récepteur étant couplé avec un composé fluorescent accepteur,

2) à ajouter, à titre de second réactif, l'analyte étant couplé avec un composé fluorescent donneur,

3) à faire incuber ledit milieu soit après l'addition de chaque réactif, soit après l'addition des deux réactifs,

4) à exciter le milieu résultant à la longueur d'onde d'excitation du composé fluorescent donneur,

5) à mesurer le signal émis par le composé fluorescent accepteur.

Dans un aspect avantageux, le premier réactif et le second réactif utilisés dans les procédés de détection et/ou de détermination par fluorescence d'un analyte indiqués ci-dessus sont ajoutés simultanément au milieu contenant l'analyte recherché.

Dans les procédés de détection et/ou de détermination par fluorescence utilisant le procédé de réduction d'interférences de l'invention, on choisira avantageusement comme composé fluorescent donneur un chélate ou un cryptate de terre rare, en particulier les cryptates décrits dans les demandes de brevets EP 180 492 et 321 353.

De préférence, le composé fluorescent donneur est le cryptate de terbium: Tb trisbipyridine ou le cryptate d'europium : Eu trisbipyridine, tels que décrits dans la demande EP 180 492.

Dans un aspect avantageux le composé fluorescent donneur est un cryptate d'europium et le composé fluorescent accepteur est choisi parmi l'allophycocyanine, l'allophycocyanine B, la C phycocyanine ou la R phycocyanine.

On peut également utiliser en tant que composé fluorescent donneur un cryptate de terbium et en tant que composé fluorescent accepteur un composé choisi parmi les rhodamines, la thionine, la R phycocyanine, la phycoerythrocyanine, la C phycoerythrine, la B phycoerythrine ou la R phycoerythrine.

L'invention sera mieux comprise à l'aide des exemples ci-après qui ne présentent aucun caractère limitatif.

Dans les exemples qui suivent, le cryptate d'europium Eu trisbipyridine diamine est préparé comme décrit dans la demande EP 321 353 (exemples 3 et 4).

Les abréviations utilisées ci-après sont les suivantes :

APC            = allophycocyanine

DTT            = dithiothréitol

EuTBP       = cryptate d'europium Eu trisbipyridine diamine

| HSA | = sérum albumine humaine |
|---|---|
| IgG | = immunoglobuline G |
| N-AANS | = acide N-acétyl 8-anilinonaphtalène 1-sulfonique |
| S-AMSA | = anhydride S-acétyl mercaptosuccinique |
| SPDP | = N-succinimidyl 3-(2-pyridyldithio)propionate |
| Sulfo-SMCC | = sulfosuccinimidyl (4-N-maléimidométhyl)cyclohexane 1-carboxylate |
| $T_4$ | = thyroxine. |

EXEMPLE 1 :

Effet du fluorure de sodium (NaF) sur la durée de vie d'émission du cryptate d'europium Eu trisbipyridine diamine en milieu sérique.

Afin d'évaluer l'effet du fluorure de sodium sur la durée de vie du signal émis par le cryptate d'europium Eu trisbipyridine diamine, on a dans un premier temps préparé un conjugué anticorps-cryptate d'europium identique aux conjugués utilisables dans un immunoessai homogène en couplant le cryptate d'europium avec un anticorps monoclonal anti-prolactine $E_1$ (CIS bio international, France). La durée de vie d'émission de ce conjugué a ensuite été mesurée en milieu tampon phosphate et en milieu sérique en présence de différentes concentrations de NaF.

A/ Préparation du conjugué anticorps-cryptate d'europium Eu trisbipyridine diamine

a) activation du cryptate Eu TBP par le sulfo-SMCC

A 5 mg (5 $10^{-6}$ moles) de Eu TBP est ajoutée une solution à 25 mM de sulfo-SMCC, en tampon phosphate 20 mM, diméthylformamide 10 % (v/v) pH 7,0 dans une proportion de 2,5 moles d'activateur par mole de Eu TBP.

Après 45 min d'activation à température ambiante, le milieu réactionnel est filtré à 0,8 $\mu$m afin d'éliminer le précipité éventuellement formé. Les produits réactionnels indésirables (sulfo-SMCC, N-hydroxysuccinimide, acide (N-maléimidométhyl)carboxylique) sont éliminés par chromatographie échangeuse d'ions sur colonne (Mono Q) (Pharmacia, Suède) en tampon phosphate 20 mM diméthylformamide 10 % (v/v), pH 7,0 sous choc de NaCl. La concentration en Eu TBP maléimide est déterminée à 307 nm avec un $\varepsilon_{307nm}$ de 25000 $M^{-1}$ $cm^{-1}$.

b) activation des IgG $E_1$ par le SPDP

Simultanément, 5 mg d'IgG $E_1$ à raison de 10 mg/ml dans un tampon phosphate 100 mM, pH 7,0 sont activés par l'ajout d'une solution de SPDP (Pierce, USA) à raison de 6,4 mM dans du dioxane dans un rapport molaire de 4 à 16 SPDP par IgG $E_1$.

Après 35 min d'activation à température ambiante, l'IgG pyridine-2 thione est purifiée sur colonne G25 superfine dans un tampon phosphate 100 mM, EDTA 5mM, pH 6,5.

Les protéines sont concentrées et les groupes 2-pyridyl disulfides sont réduits par une solution de DTT (Sigma, USA) ayant une concentration finale de 19 mM pendant 15 min à température ambiante. Le DTT et la pyridine-2-thione sont éliminés par purification sur colonne G25 superfine (Pharmacia, Suède) en tampon phosphate 100 mM, EDTA 5mM, pH 6,5. La concentration en IgG-SH est déterminée à 280 nm avec un $\varepsilon_{280nm}$ de 210000 $M^{-1}$ $cm^{-1}$.

c) Conjugaison des IgG $E_1$ avec Eu TBP-maléimide

La fixation des groupements thiols sur les maléimides est réalisée dans des proportions molaires de 10 à 30 Eu TBP maléimide par IgG $E_1$-SH. Après 18 heures d'incubation à 4°C et à l'obscurité sous agitation douce, les fonctions thiols restées libres sont bloquées par l'addition d'une solution à 100 mM de N-méthyl maléimide (Sigma, USA) ayant une concentration finale de 20 mM pendant une heure à température ambiante. Le Eu TBP non couplé est ensuite éliminée par dialyse en tampon phosphate 100 mM pH 7,0 à 4°C jusqu'à épuisement (plus de fluorescence dans les bains de dialyse).

Les caractéristiques du conjugué sont déterminées par ses absorptions à 307 nm et à 280 nm en utilisant les valeurs suivantes en tenant compte de l'absorption propre du cryptate Eu TBP déterminée par le rapport $A_{307nm}/A_{280nm}$ déterminé expérimentalement

Eu TBP-maléimide :

$\varepsilon_{307nm}$ = 25000 M$^{-1}$ cm$^{-1}$

IgG E$_1$-SH :

$\varepsilon_{280nm}$ = 210000 M$^{-1}$cm$^{-1}$

$\varepsilon_{307nm}$ = 0 M$^{-1}$cm$^{-1}$

B/ Mesure de la durée de vie d'émission du conjugué anticorps-cryptate d'europium dans l'eau et en milieu sérique en présence de NaF.

Le conjugué IgG E$_1$-Eu TBP préparé comme décrit plus haut est dilué au 1/250 dans un tampon phosphate 100 mM pH 7 contenant 1 g/l de sérum albumine humaine (HSA) et des concentrations variables de fluorure de sodium (NaF).

On ajoute 200 µl de cette solution et 100 µl de sérum de veau nouveau-né ou de tampon phosphate. On mesure ensuite la durée de vie du conjugué à 620 nm à l'aide d'un fluorimètre à laser (prototype) tel que décrit ci-après :

Un laser pulsé à azote (LASER SCIENCE INC., modèle LS1-337ND) est utilisé comme source d'excitation (longueur d'onde à 337,1 nm). La durée des pulsations est spécifiée à 3 nanosecondes et est répétée sous une fréquence de 10 Hertz. Le faisceau passe à travers un filtre (CORNING) afin d'éliminer toute lumière parasite à l'excitation autre que 337 nm.

Après être rentré dans la chambre de mesure, le faisceau est réfléchi par un filtre dichroïque, placé à 45 degrés, qui a la propriété de réfléchir les ultraviolets et de pouvoir transmettre la lumière visible.

Le faisceau réfléchi par le filtre dichroïque est focalisé sur le puits à mesurer d'une microplaque par une lentille en silice fondue. L'émission de fluorescence est collectée selon un angle solide de 20 degrés, collimatée par la même lentille, et passe directement à travers le filtre dichroïque (fluorescence en lumière visible).

Un filtre interférentiel, de caractéristiques définies selon la longueur d'onde de fluorescence à détecter, permet de se débarrasser des lumières pouvant parasiter le signal, dont l'intensité est ensuite mesurée par un photomultiplicateur (HAMAMATSU R2949).

Le compteur de photons utilisé est un SR-400 (STANFORD RESEARCH SYSTEMS), dont les opérations et la synchronisation avec le laser sont contrôlées par un ordinateur de type IBM PC-AT via une sortie RS 232 . Les pulsations provenant du photomultiplicateur sont enregistrées pendant une fenêtre de temps ($t_g$) et après un délai ($t_d$) déterminés à condition qu'elles soient supérieures à un niveau discriminant sélectionné par le compteur de photons afin d'optimiser le rapport signal/bruit du photomultiplicateur.

Une table X-Y, pilotée par l'IBM PC-AT, permet les différents positionnements de la microplaque de mesure par des moteurs pas à pas, incluant les manoeuvres de chargement, de positionnement sous le faisceau excitant, de lecture automatique en séquentiel des 96 puits, et de sortie.

On peut également utiliser un fluorimètre ARCUS (LKB, Suède) en utilisant un filtre interférentiel adapté à l'émission du composé fluorescent.

La mesure est effectuée pendant une fenêtre de temps (tg) de 100 µsec et après un délai (td) de 50 à 600 µsec.

Les résultats sont rapportés dans le tableau I ci-après:

EP 0 539 435 B1

Tableau I

| NaF   mM | $\tilde{c}$ tampon phosphate ms | $\tilde{c}$ Sérum   ms |
|---|---|---|
| 0 | 0,64 | 0,38 |
| 100 | 0,80 | 0,64 |
| 150 | 0,81 | 0,69 |
| 200 | 0,83 | 0,70 |
| 225 | 0,83 | 0,72 |

Tableau I (suite)

| 300 | 0,85 | 0,73 |
|---|---|---|
| 500 | 0,90 | 0,78 |

$\tilde{c}$ = durée de vie du signal émis.

Les résultats montrent que la durée de vie en milieu sérique est considérablement augmentée par la présence de NaF dans le milieu. A partir d'une concentration de 500 mM de NaF dans la solution de conjugué , la durée de vie du conjugué est doublée par rapport à celle du conjugué en milieu sérique sans NaF.

EXEMPLE 2 :

Effet du fluorure de potassium (KF) sur la durée de vie d'émission du cryptate d'europium Eu trisbipyridine diamine en milieu sérique.

Le conjugué IgG $E_1$-Eu TBP préparé comme décrit dans l'exemple 1 est dilué au 1/250 dans un tampon phosphate 100 mM, pH 7, contenant 1 g/l de sérumalbumine humaine (HSA) et des concentrations variables de fluorure de potassium.

On ajoute 200 µl de cette solution et 100 µl de sérum de veau nouveau-né. La mesure est réalisée dans les conditions opératoires de l'exemple 1.

Les résultats sont rapportés dans le tableau II ci-après.

7

Tableau II

| KF  mM | $\tau$ sérum ms |
|:---:|:---:|
| 0 | 0,41 |
| 100 | 0,76 |
| 200 | 0,90 |
| 400 | 1,01 |

$\tau$ = durée de vie du signal émis

Les résultats montrent que la durée de vie en milieu sérique est significativement augmentée par la présence de KF dans le milieu de mesure.

EXEMPLE 3 :

Effets de différents additifs dans différents milieux sur la durée de vie du cryptate d'europium Eu trisbipyridine diamine en présence de NaF.

Les milieux utilisés sont du sérum de veau nouveau-né et du sérum humain (pool de sera).

On utilise un conjugué IgG 3D3-Eu TBP (préparé de manière analogue au conjugué IgGE$_1$-Eu TBP de l'exemple 1 en couplant le cryptate d'europium avec l'anticorps monoclonal anti-prolactine 3D3 (CIS bio international, France) à une concentration de 0,5 µg/ml dans un tampon de dilution phosphate 100 mM, pH 7, NaF 150 mM, HSA 1g/l.

On prépare un échantillon contenant 100 µl de sérum, 100 µl de conjugué anticorps-cryptate et 100 µl de tampon contenant des concentrations variables des différents additifs suivants : EDTA, NaN$_3$ et merthiolate.

On mesure la durée de vie du conjugué anticorps-cryptate à 620 nm à l'aide d'un appareil Arcus (LKB, Suède) pendant 100 µs et après un délai de 50 à 600 µs.

Pour chaque essai, on détermine à titre de témoin les valeurs obtenues sans additif pour le conjugué anticorps-cryptate seul : l'échantillon contient alors 100 µl de sérum, 100 µl de conjugué anticorps-cryptate et 100 µl de tampon sans additif.

Les résultats obtenus sont donnés dans le tableau III ci-après :

8

Tableau III

| Additif | Concentration finale | $\tilde{c}$ ms |
|---|---|---|
| Sérum de veau nouveau-né | | |
| EDTA | 0 | 0,76 |
| | 5 g/l | 0,76 |
| | 1 g/l | 0,78 |
| | 0,5 g/l | 0,78 |
| | 0,1 g/l | 0,78 |
| NaN$_3$ | 0 | 0,76 |
| | 1 g/l | 0,75 |
| | 0,5 g/l | 0,75 |
| | 0,1 g/l | 0,75 |
| Merthiolate | 0 | 0,76 |
| | 40 mg/l | 0,79 |
| | 20 mg/l | 0,76 |
| | 10 mg/l | 0,77 |
| | 5 mg/l | 0,76 |

Tableau III (suite)

| Additif | Concentration finale | $\zeta$ ms |
|---|---|---|
| Sérum humain (pool) | | |
| EDTA | 0 | 0,65 |
| | 5 g/l | 0,71 |
| | 1 g/l | 0,67 |
| | 0,5 g/l | 0,66 |
| | 0,1 g/l | 0,66 |
| NaN$_3$ | 0 | 0,65 |
| | 5 g/l | 0,69 |
| | 1 g/l | 0,68 |
| | 0,5 g/l | 0,68 |
| | 0,1 g/l | 0,66 |
| Merthiolate | 0 | 0,65 |
| | 40 mg/l | 0,66 |
| | 20 mg/l | 0,66 |
| | 10 mg/l | 0,67 |
| | 5 mg/l | 0,66 |

Les résultats montrent que l'addition de fluorure de sodium dans le milieu de mesure permet de conserver une durée de vie du conjugué quasi constante quel que soit l'additif et sa concentration.

EXEMPLE 4 :

Dosage de la prolactine.

Un immunoessai homogène a été réalisé en utilisant comme modèle le dosage de la prolactine.

On a utilisé dans cet essai des anticorps monoclonaux anti-prolactine E$_1$ et 3D3 (CIS bio international) reconnaissant 2 épitopes disctincts de la prolactine, couplés respectivement au cryptate d'europium Eu trisbipyridine diamine et à l'allophycocyanine (Cyanotech, USA).

A) PREPARATION des IgG 3D3-APC

a) Activation de l'APC par le sulfo-SMCC

L'APC (3 mg) commercialement fournie sous forme précipitée dans une solution de 60 % de sulfate d'ammonium est centrifugée. Après élimination du surnageant, le culot est repris par 250 µl de tampon phophate 100 mM, pH 7,0, puis filtré à 0,8 µm afin d'éliminer les éventuelles particules en suspension.

Le filtrat est purifié par chromatographie d'exclusion sur colonne G25 superfine (Pharmacia, Suède) dans le même tampon. La concentration d'APC éluée dans le volume d'exclusion est déterminée à 650 nm, en considérant un $\varepsilon_{650nm}$ de 731000 M$^{-1}$ cm$^{-1}$.

L'activation de l'APC est réalisée en ajoutant une solution de sulfo-SMCC préparée extemporanément à raison de 6,9 mM dans un tampon phosphate 100 mM, pH 7,0, et en laissant la réaction se produire pendant 1 h, à température ambiante, sous agitation douce (rapport molaire de 15 à 75 sulfo-SMCC par APC). L'APC-maléimide est alors purifiée sur colonne G25 superfine en tampon phosphate 100 mM, EDTA 5 mM, pH 6,5, et conservée à 4°C avant couplage sur IgG 3D3.

b) Activation des IgG 3D3 par le SPDP

Simultanément, 5 mg d'IgG 3D3 à raison de 10 mg/ml dans un tampon phosphate 100 mM, pH 7,0, sont activés par l'ajout d'une solution de SPDP (Pierce, USA) à raison de 6,4 mM dans du dioxane dans un rapport molaire de 7,5 SPDP par IgG 3D3.

Après 35 min d'activation à température ambiante, l'IgG pyridine-2 thione est purifiée sur colonne G25 superfine dans un tampon phosphate 100 mM, EDTA 5 mM, pH 6,5.

Les protéines sont concentrées et les groupes 2-pyridyl disulfides sont réduits par une solution de DTT (Sigma, USA) ayant une concentration finale de 19 mM pendant 15 min à température ambiante. Le DTT et la pyridine-2-thione sont éliminés par purification sur colonne G25 superfine en tampon phosphate 100 mM, EDTA 5 mM, pH 6,5. La concentration en IgG-SH est déterminée à 280 nm avec un $\varepsilon_{280nm}$ de 210000 $M^{-1}cm^{-1}$.

c) Conjugaison des IgG 3D3-SH avec APC-maléimide.

La fixation des groupements thiols sur les maléimides est réalisée en ajoutant 2,51 mg d'APC activées par mg d'IgG 3D3-SH. Après 18 h d'incubation à 4°C et à l'obscurité sous agitation douce, les fonctions thiols restées libres sont bloquées par l'addition d'une solution à 100 mM de N-méthyl maléimide (Sigma, USA) ayant une concentration finale de 20 mM pendant 1 h à température ambiante.

Le milieu réactionnel est purifié par gel filtration sur colonne TSK G3000SW semi-préparative (Beckmann, USA) en tampon phosphate 100 mM, pH 7,0.

Les concentrations en APC et en IgG 3D3 du conjugué purifié, élué dans le premier pic, sont déterminées par les absorptions à 280 nm et à 650 nm, selon le calcul suivant :

$$[APC]_{mole/l} = A_{650nM}/710000$$
$$[IgG]_{mole/l} = (A_{280nm} - A'_{280nm})/210000$$

avec $A'_{280nm}$ étant la contribution à cette longueur d'onde de l'APC-maléimide, déterminée plus haut (paragraphe A - a)).

De l'albumine sérique humaine (HSA) est rajoutée à concurrence de 1 g/l au conjugué qui est ensuite réparti en aliquotes puis congelé à -20°C.

B) PREPARATION DES CONJUGUES IgG $E_1$-Eu TBP

La préparation de ces conjugués est réalisée selon le protocole décrit à l'exemple 1.

C) APPLICATION AU DOSAGE DE LA PROLACTINE

On utilise les anticorps marqués préparés comme décrit ci-dessus dans les conditions suivantes :
- IgG $E_1$ marqué avec le cryptate d'europium Eu trisbipyridine diamine à la concentration de 1 µg/ml en tampon phosphate 100 mM, NaF 150 mM, HSA 1 g/l, pH 7.
- IgG 3D3 marqué à l'allophycocyanine (Cyanotech, USA) à 2,5 µg/ml dans le même tampon.

Les standards prolactine sont réalisés dans différents milieux :
- le tampon ci-dessus
- un pool de sera humains
- une gamme standard réalisée dans du sérum de veau nouveau-né.

La prise d'essai est de 100 µl. On ajoute 100 µl de chaque conjugué et on incube 60 minutes à température ambiante dans des microplaques en polystyrène de 96 puits (Dynatech, USA).

On effectue la lecture sur un fluorimètre à laser (prototype) décrit dans l'exemple 1. Ce fluorimètre est équipé d'un filtre à 655 nm , de 20 nm de largeur à mi-hauteur en utilisant un laser pulsé à azote comme source d'excitation, dans une fenêtre de temps (tg) de 100 µs après un délai (td) de 50 µs.

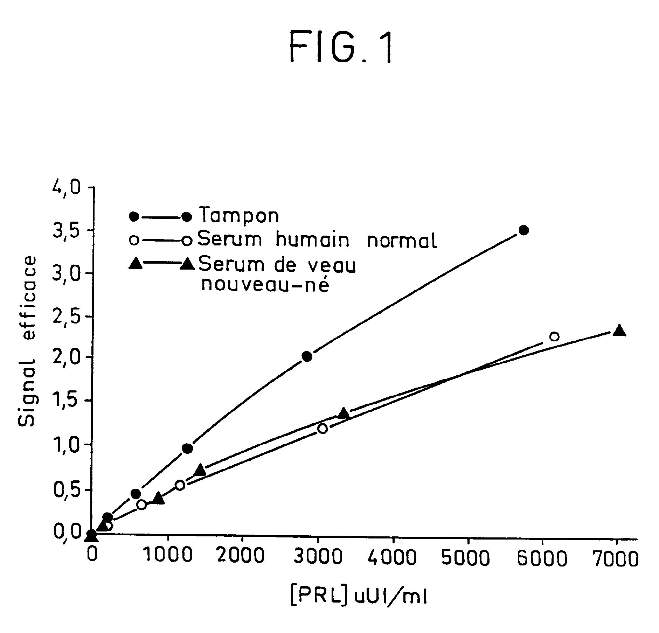

Les résultats sont représentés sur la figure 1 sur laquelle sont indiqués en abscisse la quantité de prolactine de UI/ml et en ordonnée le signal efficace $\Delta F$ déterminé par

$$\Delta F = \frac{\text{fluorescence du standard mesuré} - \text{fluorescence du standard O}}{\text{fluorescence du standard 0}}$$

Les résultats montrent que le dosage peut être effectué grâce à la présence de fluorure de sodium malgré la présence d'éléments sériques inhibiteurs de fluorescence.


EXEMPLE 5


Dosage de la prolactine

Un autre immunoessai homogène utilisant comme modèle le dosage de la prolactine a été réalisé en utilisant dans le milieu de mesure du fluorure de potassium (KF).

On utilise les anticorps marqués préparés comme décrit dans l'exemple 4 dans les conditions suivantes :
- IgG $E_1$ marqué avec le cryptate d'europium Eu trisbipyridine diamine à la concentration de 1 µg/ml en tampon phosphate 100 mM, KF 600 mM, HSA 1 g/l, pH 7.

- IgG 3D3 marqué à l'allophycocyanine (Cyanotech, USA) à 2,5 μg/l dans le même tampon.

Les standards prolactine sont réalisés dans du sérum de veau nouveau-né.

L'essai et la mesure de la fluorescence sont réalisés dans les conditions décrites dans l'exemple 4 mais en effectuant la lecture dans une fenêtre de temps (tg) de 400 μs après un délai (td) de 50 μs.

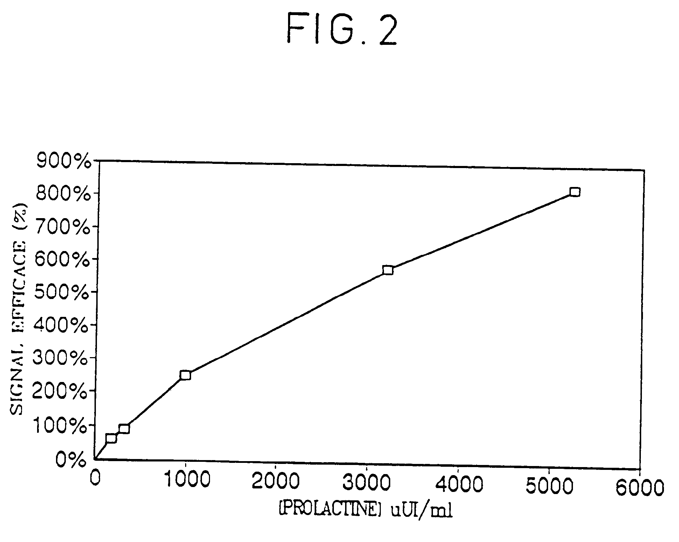

Les résultats sont donnés sur la figure 2 sur laquelle sont indiqués en abscisse la quantité de prolactine en UI/ml et en ordonnée le signal efficace déterminé comme dans l'exemple 4.

Les résultats montrent que la présence du fluorure de potassium dans le milieu assure la sensibilité du dosage malgré la présence d'éléments sériques inhibiteurs de fluorescence.

EXEMPLE 6 :

Dans un essai analogue à celui qui a été décrit dans l'exemple 2 ci-dessus, l'effet du fluorure de sodium à une concentration finale de 100 mM a également été testé sur 50 sera humain en utilisant un conjugué anticorps 3D3 - cryptate Eu trisbipyridine.

Les résultats obtenus montrent que la durée de vie du conjugué anticorps-cryptate est constante, contrairement à ce qui est observé dans la pratique dans ce type de dosage en milieu sérique.

EXEMPLE 7 :

Dosage de la digoxine

La digoxine est un glucoside cardiotonique utilisé comme principe actif de médicaments traitant l'insuffisance cardiaque.

A/ Préparation d'un traceur allophycocyanine-digoxine par couplage au periodate.

a) activation de la digoxine par le periodate

268 μl d'une solution de $NaIO_4$ (Fluka, Suisse) préparée extemporanément sont ajoutés à une suspension de digoxine (ref 37100 Fluka, Suisse) de 5,35 mg dans 268 μl d'éthanol absolu.

Le mélange est incubé pendant 20 min à température ambiante sous agitation, puis la réaction est bloquée par ajout de 100 μl de glycérol 0,1 M.

La concentration finale en digoxine activée calculée sur la masse initiale et le volume final est de $1,2 \times 10^{-2}$M.

b) couplage de la digoxine activée avec l'allophycocyanine (APC)

A 2 ml de solution APC purifiée (Cyanotech, USA) en tampon borate pH 9,0 sont ajoutés 95 μl de la solution de digoxine activée au periodate.

Le mélange est incubé pendant 1h30 à température ambiante sous agitation douce. La réaction est bloquée par ajout de 100 μl d'une solution de borohydrure de sodium préparée extemporanément à raison de 5 mg de $NaBH_4$ dans 1,32 ml de tampon borate pH 9,0.

c) purification du traceur APC-digozine

2 ml du mélange réactionnel sont injectés sur une colonne HR 10/10 G25 (Pharmacia, Suède) équilibrée en tampon phosphate 100 mM, pH 7, et élués dans le volume exclu. Les réactifs en excès sont élués par 8 ml de tampon.

On récupère environ 4 ml de solution de produit couplé APC-digoxine à 1,22 mg/ml en APC (mesurée par l'absorbance à 650 nm).

La concentration en digoxine est évaluée par un dosage RIA ou DELFIA (Pharmacia, Suède).

Le rapport molaire final calculé digoxine/allophycocyanine est d'environ 0,8.

B/ Dosage homogène compétitif de la digoxine en sérum humain.

On prépare un conjugué anticorps-cryptate d'europium Eu TBP diamine de manière analogue à celle décrite dans l'exemple 1, en utilisant un anticorps monoclonal de souris anti-digoxine (ref. G 31604 M, Interchim, France). Ce conjugué anticorps-cryptate est utilisé à la concentration de 0,5 μg/ml en tampon phosphate 100 mM, NaF 150 mM, HSA 1 g/l, pH 7.

Le traceur APC-digoxine est utilisé à une concentration de 0,2 μg/ml dans le même tampon.

On prépare une gamme de standards digoxine par dilution en sérum humain d'une solution de digoxine (Fluka, Suisse) à 1 mg/ml dans un mélange éthanol/eau 50/50.

La courbe standard est réalisée à l'aide de prises de 250 μl contenant

- 50 μl de tampon phosphate
- 50 μl de standard
- 100 μl de traceur APC-digoxine
- 100 μl de conjugué anticorps-cryptate.

Les échantillons à doser sont composés de la même façon en remplaçant les 50 µl de standard par 50 µl de sérum individuel à doser.

Le dosage est réalisé dans des microplaques de 96 puits (Dynatech, USA).

Après 30 min d'incubation à température ambiante, on effectue la lecture sur un fluorimètre à laser tel que décrit dans l'exemple 1, équipé d'un filtre à 665 nm, de 20 nm de largeur à mi-hauteur, dans une fenêtre de temps de 100 µs, après un délai de 50 µs.

Les résultats sont exprimés en pourcentage du rapport $B/B_o$ dans lequel B représente la valeur obtenue pour chaque standard et $B_o$ celle du standard 0.

Les résultats obtenus sont rapportés dans le tableau IV ci-après :

TABLEAU IV

| Standard digoxine ng/ml | $B/B_o$ % |
|---|---|
| 0 | 100 |
| 1,6 | 96,21 |
| 3,2 | 87,15 |
| 6,4 | 73,57 |
| 12,8 | 56,24 |
| 26 | 48,86 |

Ces résultats montrent que les valeurs obtenues varient proportionnellement à la concentration en digoxine.La présence de fluorure de sodium dans le milieu de mesure permet d'effectuer le dosage de la digoxine en milieu sérique avec une sensibilité de l'ordre du ng/ml.

EXEMPLE 8 :

Dosage de la thyroxine.
A/ Préparation d'un traceur allophycocyanine-thyroxine (APC-$T_4$)
a) Activation de la thyroxine par le S-AMSA
A 500 µl d'une solution à 20 mg/ml de $T_4$ (Calbiochem, France) dans du méthanol sont ajoutés 500 µl d'une solution de S-AMSA (Sigma, USA) à 8,7 mg/ml (50mM) dans du méthanol et le mélange est incubé 15 min à température ambiante, avant ajout de 500 µl d'une solution d'hydroxylamine à 6,95 mg/ml (100 mM) dans du méthanol. Après 15 min d'incubation à température ambiante, 975 µl du mélange réactionnel sont prélevés et additionnés de 525 µl d'eau bidistillée filtrée sur filtre MILLIPORE HA 0,45 µm. Cette solution est passée par fractions de 250 µl sur une colonne HPLC Pep.RPC HR 5/5 (Pharmacia, Suède) et éluée par un mélange méthanol/eau 65/35. Les trois premiers pics élués sont récupérés, et les fractions successives sont jointes et évaporées sous vide dans un évaporateur rotatif. On récupère environ 3 mg de $T_4$ activée ($T_4$-SH).
b) Activation de l'allophycocyanine par le SMCC
10 mg d'APC sont purifiés, puis concentrés sur cône AMICON CENTRICON 30 jusqu'à une concentration de 10,6 mg/ml sous 700 µl.
A cette solution sont ajoutés 160 µl d'une solution de sulfo-SMCC (Pierce, USA) à 10 mg/ml dans un tampon phosphate 100 mM, pH 7. Le mélange réactionnel est incubé 1 h à température ambiante sous agitation, puis l'APC activée est purifiée par chromatographie d'exclusion sur colonne G 25 HR 10/10 (Pharmacia, Suède) en tampon phosphate pH 7.
c) Couplage de la thyroxine activée avec l'allophycocyanine activée
Le contenu du ballon de $T_4$-SH est repris par 200 µl de méthanol 50 µl de cette solution sont ajoutés à 2 ml de solution d'APC activée en tampon phosphate 100 mM pH 7. Le mélange réactionnel est incubé

13

18 h à 4°C sous agitation, puis les sites maléimides en excès sont bloqués par 5 µl d'une solution de mercaptoéthanol (Sigma, USA) au 1/10 dans le tampon phosphate.

d) Purification du traceur APC-$T_4$

Le mélange réactionnel est concentré jusqu'à un volume de 1 ml sur dispositif d'ultrafiltration AMICON CENTRICON 30, puis 50 µl d'une solution de N-AANS à 1 mg/ml dans le tampon phosphate sont ajoutés (concentration finale en N-AANS environ 50 g/ml). Le traceur est finalement purifié sur colonne de chromatographie d'exclusion PHARMACIA G 25 HR 10/10 (tampon d'élution phosphate 100 mM pH 7). On obtient 2,5 ml environ de traceur à la concentration (calculée par DO à 650 nm, $\varepsilon$ = 731 000) de 0,8 mg/ml.

L'évaluation du nombre de molécules de $T_4$ couplées par molécule d'APC est réalisée par dosage RIA du traceur et comparaison à une courbe standard en $T_4$ (trousse $T_4$-KPR, ORIS Industrie, France). Le calcul donne un rapport sur le traceur purifié de 1,4 $T_4$/APC.

La purification du traceur APC-$T_4$ s'effectue en présence de N-AANS synthétisé à partir de N-ANS (KODAK, USA) par une modification du protocole décrit par HINDS et al. (CLIN. CHEM. 32, 16-21, 1986).

B/ Dosage homogène compétitif de la thyroxine en sérum humain

On prépare un conjugué anticorps-cryptate d'europium Eu TBP diamine de manière analogue à celle décrite dans l'exemple 1, en utilisant un anticorps monoclonal de souris anti-$T_4$ $R_{41}$ (CIS bio international, France). Ce conjugué est utilisé à la concentration de 2 g/ml en tampon phosphate 100 mM, NaF 120 mM, HSA 0,2 %, pH 7.

On prépare une gamme de standards $T_4$ par dilution dans un sérum humain normal traité par échange d'ions et dépourvu de $T_4$. La courbe standard est réalisée à l'aide de prises de 250 µl contenant

- 50 µl de solution de N-AANS
- 50 µl de sérum humain dépourvu de $T_4$ ou 50 µl de l'un des standards $T_4$
- 100 µl de traceur APC-$T_4$
- 100 µl de conjugué anticorps-cryptate.

Les échantillons à doser sont composés de la même manière, en remplaçant les 50 µl de standard qui sont remplacés par 50 µl de sérum individuel à doser.

Le dosage est réalisé dans des microplaques de 96 puits (Dynatech, USA).

Après incubation pendant 30 min à température ambiante, on effectue la lecture sur un fluorimètre à laser tel que décrit dans l'exemple 1 équipé d'un filtre à 665 nm, de 20 nm de largeur à mi-hauteur, en utilisant une lampe Flash 1000 Hz comme source d'excitation pendant 1 s, dans une fenêtre de temps de 100 µs, après un délai de 50 µs.

Les valeurs obtenues pour chaque standard (B) sont divisées par la valeur du standard 0 ($B_o$) et exprimées en pourcentage (B/$B_o$ %). La concentration des échantillons à doser est calculée par comparaison avec la courbe standard.

Les résultats sont rapportés dans le tableau V ci-après:

TABLEAU V

| thyroxine ng/ml | B/$B_o$ % |
|---|---|
| 0 | 100 |
| 10 | 91 |
| 20 | 82,6 |
| 50 | 61,7 |
| 100 | 43,3 |
| 250 | 23 |

Les résultats montrent que les valeurs obtenues varient proportionnellement à la concentration en

14

$T_4$. La présence de fluorure de sodium dans le milieu de mesure permet d'assurer la sensibilité de la mesure en milieu sérique.

EXEMPLE 9 :

Dosage de l'antigène 19.9.

L'antigène 19.9 est un carbohydrate représentatif du carcinome du colon.

Le site de l'anticorps anti-19.9 étant un épitope répétitif, on marque le même anticorps soit avec le donneur, soit avec l'accepteur.

On utilise pour cet immuno-essai homogène des conjugués anticorps - Eu TBP et anticorps -APC préparés de manière analogue à celle décrite dans l'exemple 4.

L'antigène 19.9 et l'anticorps anti-19.9 sont fournis par Centochor, USA.

Les deux conjugués sont dilués dans un tampon phosphate 100 mM, Na F 150 mM, HSA 1 g/l, pH 6.

On réalise une gamme standard d'antigène 19.9 par dilution d'une solution concentrée d'antigène dans du sérum de veau nouveau-né.

On ajoute successivement dans des microplaques en polystyrène de 96 puits (Dynatech, USA) :
- 50 μl de solution standard d'antigène 19.9
- 50 μl de tampon de dilution
- 100 μl de conjugué anticorps-Eu TBP à 0,5 μg/ml
- 100 μl de conjugué anticorps-APC à 5 μg/ml.

Après incubation pendant 3 h30 à température ambiante, on effectue la lecture à l'aide du fluorimètre à laser décrit dans l'exemple 1 équipé d'un filtre à 650 nm de 20 nm de largeur, avec un délai de 50 μs pendant 400 μs.

Les résultats sont rapportés dans le tableau VI ci-après et exprimés en unités arbitraires (UA).

TABLEAU VI

| Antigène 19.9 u/ml | Signal UA |
|---|---|
| 0 | 312 |
| 9 | 419 |
| 30 | 572 |
| 64 | 890 |
| 138 | 1602 |
| 278 | 2491 |

Ces résultats montrent que le signal mesuré varie proportionnellement à la quantité d'antigène 19.9 dans le milieu.

La présence du fluorure de sodium dans le milieu de mesure permet à nouveau d'effectuer la mesure avec une grande sensibilité.

EXEMPLE 10 :

Dosage de l'antigène carcinoembryonnaire (ACE).

Dans cet immuno-essai homogène, on utilise deux anticorps monoclonaux G12 et G15 (CIS bio international, France) couplés respectivement avec du cryptate d'europium Eu TBP et de l'allophycocyanine.

Les deux conjugués G12-Eu TBP et G15-APC sont dilués dans un tampon phosphate 100 mM, HSA 1 g/l, NaF 150 mM.

On ajoute successivement dans des microplaques en polystyrène (Dynatech, USA):
- 100 µl de solution standard
- 100 µl de conjugué G12-cryptate à 0,5 µg/ml
- 100 µl de conjugué G15-APC à 5 µg/ml.

Après incubation pendant 3 h à 37°C on effectue la lecture à l'aide d'un fluorimètre à laser décrit dans l'exemple 1 équipé d'un filtre à 650 nm de 20 nm de largeur, avec un délai de 50 µs, pendant 400 µs.

Les résultats obtenus sont rapportés dans le tableau VII ci-après et exprimés en unités arbitraires (UA) :

TABLEAU VII

| ACE<br>ng/ml | Signal<br>UA |
|---|---|
| 0 | 345 |
| 5,9 | 420 |
| 21 | 474 |
| 72 | 769 |
| 2634 | 1058 |

Les résultats montrent une variation du signal émis proportionnelle à la concentration en ACE. De plus, l'amplification du signal permet de détecter l'ACE avec une sensibilité de l'ordre du ng/ml.

La présence du fluorure de sodium dans le milieu de mesure permet à nouveau d'effectuer la mesure avec une grande sensibilité.

**Revendications**

1.  Procédé de réduction d'interférences dans un dosage par fluorescence d'un analyte utilisant comme traceur au moins un composé fluorescent, caractérisé en ce qu'on ajoute au milieu de mesure des ions fluorures.

2.  Procédé selon la revendication 1, caractérisé en ce qu'on utilise en tant que traceur fluorescent un chelate ou un cryptate de terre rare.

3.  Procédé selon les revendications 1 ou 2 , caractérisé en ce que le milieu de mesure est un milieu biologique.

4.  Procédé selon la revendication 3, caractérisé en ce que le milieu de mesure est un milieu sérique.

5.  Procédé selon les revendications 3 ou 4, caractérisé en ce qu'on ajoute au milieu de mesure du fluorure de sodium, de cesium, de potassium, ou de lithium.

6.  Procédé selon la revendication 5, caractérisé en ce que le milieu de mesure est un milieu sérique et en ce que la concentration en fluorure de sodium est de 50 à 500 mM/l.

7.  Procédé selon la revendication 5, caractérisé en ce que le milieu de mesure est un milieu sérique et en ce que la concentration en fluorure de potassium est de 50 mM/l à 5 M/l.

8.  Utilisation des ions fluorures pour réduire les interférences dans le milieu de mesure d' un procédé de détection et/ou de détermination par fluorescence d'un analyte dans un milieu susceptible de le contenir.

9. Utilisation selon la revendication 8, dans un procédé homogène de détection et/ou de détermination par fluorescence d'un analyte dans un milieu susceptible de le contenir.

10. Utilisation selon l'une quelconque des revendications 8 ou 9, dans un procédé de détection et/ou de détermination par fluorescence d'un analyte dans un milieu susceptible de le contenir consistant :
1) à ajouter audit milieu un premier réactif constitué d'au moins un récepteur dudit analyte,
2) à ajouter un second réactif choisi parmi l'analyte ou au moins l'un de ses récepteurs, l'un des deux réactifs étant couplé avec un composé fluorescent donneur et l'autre réactif étant couplé avec un composé fluorescent accepteur et l'ordre d'ajout des réactifs pouvant être inversé,
3) à faire incuber le milieu résultant soit après l'addition de chaque réactif, soit après l'addition des deux réactifs,
4) à exciter le milieu résultant à la longueur d'onde d'excitation du composé donneur,
5) à mesurer, à l'équilibre ou en cinétique, le signal émis par le composé fluorescent accepteur.

11. Utilisation selon la revendication 10, dans un procédé de détection et/ou de détermination par fluorescence d'un analyte dans un milieu susceptible de le contenir à l'aide d'une méthode par excès consistant :
1) à ajouter, audit milieu contenant l'analyte recherché, un premier réactif constitué par au moins un récepteur dudit analyte, couplé avec un composé fluorescent donneur,
2) à ajouter un second réactif constitué par un ou plusieurs autres récepteurs dudit analyte, ledit second réactif étant couplé avec un composé fluorescent accepteur,
3) à faire incuber ledit milieu après chaque addition de réactifs ou après l'addition des deux réactifs,
4) à exciter le milieu résultant à la longueur d'onde d'excitation du composé fluorescent donneur par une source de lumière,
5) à mesurer le signal émis par le composé fluorescent accepteur.

12. Utilisation selon la revendication 10, dans un procédé de détection et/ou de détermination par fluorescence d'un analyte dans un milieu susceptible de le contenir à l'aide d'une méthode par compétition consistant :
1) à ajouter, audit milieu contenant l'analyte recherché, un premier réactif constitué par un récepteur dudit analyte, couplé avec un composé fluorescent donneur,
2) à ajouter un second réactif constitué de l'analyte couplé avec un composé fluorescent accepteur,
3) à faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent accepteur.

13. Utilisation selon la revendication 10, dans un procédé de détection et/ou de détermination par fluorescence d'un analyte dans un milieu susceptible de le contenir à l'aide d'une méthode par compétition consistant :
1) à ajouter, audit milieu contenant l'analyte recherché, un premier réactif constitué par un récepteur dudit analyte, ledit récepteur étant couplé avec un composé fluorescent accepteur,
2) à ajouter, à titre de second réactif, l'analyte étant couplé avec un composé fluorescent donneur,
3) à faire incuber ledit milieu soit après l'addition de chaque réactif, soit après l'addition des deux réactifs,
4) à exciter le milieu résultant à la longueur d'onde d'excitation du composé fluorescent donneur,
5) à mesurer le signal émis par le composé fluorescent accepteur.

14. Utilisation selon l'une quelconque des revendications 10 à 13, caractérisé en ce que le premier réactif et le second réactif sont ajoutés simultanément au milieu contenant l'analyte recherché.

15. Utilisation selon l'une quelconque des revendications 10 ou 11, caractérisé en ce qu'on utilise un seul récepteur de l'analyte, qui est couplé soit avec le composé luminescent donneur, soit avec le composé luminescent accepteur.

16. Utilisation selon l'une quelconque des revendications 10 à 15, dans laquelle le composé fluorescent donneur est un chélate ou cryptate de terre rare.

17. Utilisation selon la revendication 16, dans laquelle le cryptate de terre rare est un cryptate de terbium ou d'europium.

18. Utilisation selon la revendication 17, caractérisée en ce que le composé donneur est le cryptate de terbium Tb trisbipyridine ou le cryptate d'europium Eu trisbipyridine.

19. Utilisation selon l'une quelconque des revendications 10 à 16, caractérisée en ce que le composé fluorescent donneur est un cryptate d'europium et en ce que le composé fluorescent accepteur est choisi parmi l'allophycocyanine, l'allophycocyanine B, la C phycocyanine ou la R phycocyanine.

20. Utilisation selon l'une quelconque des revendications 10 à 16, caractérisée en ce que le composé fluorescent donneur est un cryptate de terbium et en ce que le composé fluorescent accepteur est choisi parmi les rhodamines, la thionine, la R phycocyanine, la phycoerythrocyanine, la C phycoerythrine, la B phycoerythrine ou la R phycoerythrine.

## Patentansprüche

1. Verfahren zur Verminderung von Störungen bei fluorimetrischen Bestimmungen einer zu analysierenden Substanz, wobei als Tracer mindestens eine fluoreszierende Verbindung verwendet wird, dadurch gekennzeichnet, daß dem Meßmedium Fluoridionen zugesetzt werden.

2. Verfahren nach Anspruch 1, dadurch gekennzeichnet, daß als fluoreszierender Tracer ein Seltenerdmetallchelat oder ein Seltenerdmetallkryptat verwendet wird.

3. Verfahren nach Anspruch 1 oder 2, dadurch gekennzeichnet, daß das Meßmedium ein biologisches Medium ist.

4. Verfahren nach Anspruch 3, dadurch gekennzeichnet, daß das Meßmedium ein Serummedium ist.

5. Verfahren nach Anspruch 3 oder 4, dadurch gekennzeichnet, daß dem Meßmedium Natriumfluorid, Cäsiumfluorid, Kaliumfluorid oder Lithiumfluorid zugesetzt werden.

6. Verfahren nach Anspruch 5, dadurch gekennzeichnet, daß das Meßmedium ein Serummedium ist und die Konzentration an Natriumfluorid 50 bis 500 mM beträgt.

7. Verfahren nach Anspruch 5, dadurch gekennzeichnet, daß das Meßmedium ein Serummedium ist und die Konzentration an Kaliumfluorid 50 mM bis 5 M beträgt.

8. Verwendung von Fluoridionen zur Verminderung von Störungen im Meßmedium bei Verfahren zur qualitativen und/oder quantitativen fluorimetrischen Bestimmung einer zu analysierenden Substanz in einem Medium, in dem sie enthalten sein kann

9. Verwendung nach Anspruch 8 bei einem homogenen Verfahren zur qualitativen und/oder quantitativen fluorimetrischen Bestimmung einer zu analysierenden Substanz in einem Medium, in dem sie enthalten sein kann.

10. Verwendung nach Anspruch 8 oder 9 bei einem Verfahren zur qualitativen und/oder quantitativen fluorimetrischen Bestimmung einer zu analysierenden Substanz in einem Medium, in dem sie enthalten sein kann, das folgende Schritte umfaßt:
    1) Zugabe eines ersten Reaktanten, der aus mindestens einem Rezeptor der zu analysierenden Substanz besteht, zum Medium,
    2) Zugabe eines zweiten, unter der zu analysierenden Substanz und mindestens einem ihrer Rezeptoren ausgewählten Reaktanten, wobei einer der beiden Reaktanten an eine fluoreszierende Donorverbindung und der andere Reaktant an eine fluoreszierende Rezeptorverbindung gekuppelt sind, wobei die Reihenfolge der Zugabe der Reaktanten umgekehrt werden kann,
    3) Inkubation des resultierenden Mediums nach Zugabe jedes Reaktanten oder nach Zugabe beider Reaktanten,
    4) Anregung des resultierenden Mediums bei der Anregungswellenlänge der Donorverbindung und
    5) Messung des von der fluoreszierenden Akzeptorverbindung emittierten Signals im Gleichgewicht oder kinetisch.

11. Verwendung nach Anspruch 10 bei einem Verfahren zur qualitativen und/oder quantitativen fluorimetrischen Bestimmung einer zu analysierenden Substanz in einem Medium, in dem sie enthalten sein kann, nach einem Überschußverfahren, das folgende Schritte umfaßt:

1) Zugabe eines ersten Reaktanten, der aus mindestens einem Rezeptor der zu analysierenden Substanz besteht, der an eine fluoreszierende Donorverbindung gekuppelt ist, zu dem die zu analysierende Substanz enthaltenden Medium,

2) Zugabe eines zweiten, aus einem oder mehreren anderen Rezeptoren der zu analysierenden Substanz bestehenden Reaktanten, wobei der zweite Reaktant an eine fluoreszierende Akzeptorverbindung gekuppelt ist,

3) Inkubation des Mediums nach jeder Zugabe von Reaktanten oder nach Zugabe beider Reaktanten,

4) Anregung des resultierenden Milieus bei der Anregungswellenlänge der fluoreszierenden Donorverbindung mit einer Lichtquelle und

5) Messung des von der fluoreszierenden Akzeptorverbindung emittierten Signals.

12. Verwendung nach Anspruch 10 bei einem Verfahren zur qualitativen und/oder quantitativen fluorimetrischen Bestimmung einer zu analysierenden Substanz in einem Medium, in dem sie enthalten sein kann, nach einem kompetitiven Verfahren, das folgende Schritte umfaßt:

1) Zugabe eines ersten Reaktanten, der aus einem Rezeptor der zu analysierenden Substanz besteht, der an eine fluoreszierende Donorverbindung gekuppelt ist, zu dem Medium, das die zu analysierende Substanz enthält,

2) Zugabe eines zweiten Reaktanten, der aus der an eine fluoreszierende Akzeptorverbindung gekuppelten zu analysierenden Substanz besteht,

3) Inkubation des Mediums nach jeder Zugabe von Reaktanten oder nach Zugabe beider Reaktanten,

4) Anregung des resultierenden Mediums bei der Anregungswellenlänge der fluoreszierenden Donorverbindung und

5) Messung des von der fluoreszierenden Akzeptorverbindung emittierten Signals.

13. Verwendung nach Anspruch 10 bei einem Verfahren zur qualitativen und/oder quantitativen fluorimetrischen Bestimmung einer zu analysierenden Substanz in einem Medium, in dem sie enthalten sein kann, nach einem kompetitiven Verfahren, das folgende Schritte umfaßt:

1) Zugabe eines ersten Reaktanten, der aus einem Rezeptor der zu analysierenden Substanz besteht, der an eine fluoreszierende Akzeptorverbindung gekuppelt ist, zu dem Medium, das die zu analysierende Substanz enthält,

2) Zugabe der an eine fluoreszierende Donorverbindung gekuppelten zu analysierenden Substanz als zweiten Reaktanten,

3) Inkubation des Mediums nach Zugabe jedes Reaktanten oder nach Zugabe beider Reaktanten,

4) Anregung des resultierenden Mediums bei der Anregungswellenlänge der fluoreszierenden Donorverbindung und

5) Messung des von der fluoreszierenden Akzeptorverbindung emittierten Signals.

14. Verwendung nach einem der Ansprüche 10 bis 13, dadurch gekennzeichnet, daß der erste und der zweite Reaktant dem die zu analysierende Substanz enthaltenden Medium gleichzeitig zugegeben werden.

15. Verwendung nach Anspruch 10 oder 11, dadurch gekennzeichnet, daß ein einziger Rezeptor der zu analysierenden Verbindung verwendet wird, der entweder an die fluoreszierende Donorverbindung oder an die fluoreszierende Akzeptorverbindung gekuppelt ist.

16. Verwendung nach einem der Ansprüche 10 bis 15, wobei die fluoreszierende Donorverbindung ein Seltenerdmetallchelat oder ein Seltenerdmetallkryptat ist.

17. Verwendung nach Anspruch 16, wobei das Seltenerdmetallkryptat ein Terbium- oder Europiumkryptat ist.

18. Verwendung nach Anspruch 17, dadurch gekennzeichnet, daß die Donorverbindung das Terbiumkryptat Tb-Tris(bipyridin) oder das Europiumkryptat Eu-Tris(bipyridin) ist.

19. Verwendung nach einem der Ansprüche 10 bis 16, dadurch gekennzeichnet, daß die fluoreszierende Donorverbindung ein Europiumkryptat ist und die fluoreszierende Akzeptorverbindung unter Allophycocyanin, Allophycocyanin B, C-Phycocyanin und R-Phycocyanin ausgewählt ist.

20. Verwendung nach einem der Ansprüche 10 bis 16, dadurch gekennzeichnet, daß die fluoreszierende Donorverbindung ein Terbiumkryptat ist und die fluoreszierende Akzeptorverbindung unter den Rhodaminen, Thionin, R-Phycocyanin, Phycoerythrocyanin, C-Phycoerythrin, B-Phycoerythrin und R-Phycoerythrin ausgewählt ist.

## Claims

1. Method of reducing interference in a fluorescent assay of an analyte using at least one fluorescent compound as the tracer, characterized in that fluoride ions are added to the measuring medium.

2. Method according to claim 1, characterized in that the fluorescent tracer used is a rare earth chelate or cryptate.

3. Method according to claims 1 or 2, characterized in that the measuring medium is a biological medium.

4. Method according to claim 3, characterized in that the measuring medium is a serum medium.

5. Method according to claims 3 or 4, characterized in that sodium, cesium, potassium or lithium fluoride is added to the measuring medium.

6. Method according to claim 5, characterized in that the measuring medium is a serum medium and in that the concentration of sodium fluoride is 50 to 500 mM/l.

7. Method according to claim 5, characterized in that the measuring medium is a serum medium and in that the concentration of potassium fluoride is 50 mM/l to 5 M/l.

8. Use of fluoride ions for reducing interference in the measuring medium of a fluorescent method of detecting and/or determining an analyte in a medium which may contain it.

9. Use according to claim 8, in a homogeneous fluorescent method of detecting and/or determining an analyte in a medium which may contain it.

10. Use according to any one of claims 8 or 9, in a fluorescent method of detecting and/or determining an analyte in a medium which may contain it, consisting in:
    1) adding to said medium a first reagent constituted of at least one receptor for said analyte,
    2) adding a second reagent selected from the analyte or at least one of its receptors, one of the two reagents being coupled with a fluorescent donor compound and the other reagent being coupled with a fluorescent acceptor compound, and the order of addition of the reagents being reversible,
    3) incubating the resulting medium either after the addition of each reagent or after the addition of both reagents,
    4) exciting the resulting medium at the excitation wavelength of the donor compound, and
    5) measuring, at equilibrium or under kinetic conditions, the signal emitted by the fluorescent acceptor compound.

11. Use according to claim 10 in a fluorescent method of detecting and/or determining an analyte in a medium which may contain it, with the aid of an excess method consisting in:
    1) adding, to said medium containing the analyte in question, a first reagent consisting of at least one receptor for said analyte, coupled with a fluorescent donor compound,
    2) adding a second reagent consisting of one or more other receptors for said analyte, said second reagent being coupled with a fluorescent acceptor compound,
    3) incubating said medium either after the addition of each reagent or after the addition of both reagents,
    4) exciting the resulting medium at the excitation wavelength of the fluorescent donor compound by means of a light source, and
    5) measuring the signal emitted by the fluorescent acceptor compound.

12. Use according to claim 10 in, a fluorescent method of detecting and/or determining an analyte in a medium which may contain it, with the aid of a competitive method consisting in:
    1) adding, to said medium containing the analyte in question, a first reagent consisting of a receptor for said analyte, coupled with a fluorescent donor compound,

2) adding a second reagent made up of the analyte coupled with a fluorescent acceptor compound,

3) incubating said medium either after the addition of each reagent or after the addition of both reagents,

4) exciting the resulting medium at the excitation wavelength of the fluorescent donor compound, and

5) measuring the signal emitted by the fluorescent acceptor compound.

13. Use according to claim 10, in a fluorescent method of detecting and/or determining an analyte in a medium which may contain it, with the aid of a competitive method consisting in:

1) adding, to said medium containing the analyte in question, a first reagent consisting of a receptor for said analyte, said receptor being coupled with a fluorescent acceptor compound,

2) adding, as a second reagent, the analyte being coupled with a fluorescent donor compound,

3) incubating said medium either after the addition of each reagent or after the addition of both reagents,

4) exciting the resulting medium at the excitation wavelength of the fluorescent donor compound, and

5) measuring the signal emitted by the fluorescent acceptor compound.

14. Use according to any one of claims 10 to 13, characterized in that the first reagent and second reagent are added simultaneously to the medium containing the analyte in question.

15. Use according to claims 10 or 11, characterized in that the single receptor for the analyte is used which is coupled either with the luminescent donor compound or with the luminescent acceptor compound.

16. Use according to any one of claims 10 to 15, in which the fluorescent donor compound is a rare earth chelate or cryptate.

17. Use according to claim 16, in which the rare earth cryptate is a terbium or europium cryptate.

18. Use according to claim 17, characterized in that the donor compound is the terbium cryptate Tb trisbipyridine or the europium cryptate Eu trisbipyridine.

19. Use according to any one of claims 10 to 16, characterized in that the fluorescent donor compound is a europium cryptate and in that the fluorescent acceptor compound is selected from allophycocyanin, allophycocyanin B, phycocyanin C or phycocyanin R.

20. Use according to any one claims 10 to 16, characterized in that the fluorescent donor compound is a terbium cryptate and in the fluorescent acceptor compound is selected from rhodamines, thionine, phycocyanin R, phycoerythrocyanin, phycoerythrin C, phycoerythrin B or phycoerythrin R.

# FIG.1

# FIG. 2